(19) Europäisches Patentamt
European Patent Office
Office européen des brevets

(11) EP 0 956 855 B1

(12) EUROPEAN PATENT SPECIFICATION

(45) Date of publication and mention of the grant of the patent:
**12.03.2003 Bulletin 2003/11**

(21) Application number: **98201323.7**

(22) Date of filing: **24.04.1998**

(51) Int Cl.[7]: **A61K 31/522**, C07D 473/06, C07D 473/10, A61K 31/453, A61K 38/13, A61K 31/436, A61K 31/42, A61K 31/365, A61P 37/00
// (A61K31/522, 31:453), (A61K38/13, 31:522), (A61K31/522, 31:436), (A61K31/522, 31:42), (A61K31/522, 31:365)

# (54) Immunosuppressive effects of 8 substituted xanthine derivatives

Immununterdrückende Effekte von 8 substituierten Xanthinderivaten

Effets immunosuppresseurs des xanthines 8 substituées

(84) Designated Contracting States:
**AT BE CH DE DK ES FR GB GR IT LI LU NL SE**

(43) Date of publication of application:
**17.11.1999 Bulletin 1999/46**

(73) Proprietors:
- **K.U. Leuven Research & Development**
  **3000 Leuven (BE)**
- **Pfleiderer, Wolfgang**
  **78464 Konstanz (DE)**

(72) Inventors:
- **Waer, Mark Jozef Albert**
  **3001 Heverlee (BE)**
- **Herdewijn, Piet André Maurits Maria**
  **3111 Rotzelaar/Wezemaal (BE)**
- **Pfleiderer, Wolfgang**
  **78464 Konstanz (DE)**

(74) Representative: **Bartelds, Erik**
**Arnold & Siedsma,**
**Advocaten en Octrooigemachtigden,**
**Sweelinckplein 1**
**2517 GK Den Haag (NL)**

(56) References cited:
**EP-A- 0 490 181** **WO-A-96/36638**

- **M.H. HOLSCHBACH ET AL.: "A1 adenosine receptor antagonists as ligands for positron emission tomography and single-photon emission tomography." J. MED. CHAM, vol. 41, no. 4, 1998, pages 555-563, XP002078914**
- **I.A. DOICHINOVA ET AL.: "QSAR studies of 8-substituted xanthines as adenosne receptor antagonists." EUR. J. MED. CHEM., vol. 29, no. 2, 1994, pages 133-138, XP000574700**
- **C.E. MÜLLER ET AL.: "Synthesis of paraxanthine analogs (1,7-disubstituted xanthines) and other xanthines unsubstituted at the 3-position: SAR at adenosine receptor." J. MED. CHEM., vol. 36, no. 22, 1993, pages 3341-3349, XP000574701**
- **M.T. SHAMIN ET AL.: "Effects of 8-phenyl and 8-cyclalkyl substituents on the activity of mono-, di- and trisubstituted alkylxanthnes with substitution at the 1-, 3- and 7-positions." J. MED. CHEM., vol. 32, no. 6, 1989, pages 1231-1237, XP002078917**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 956 855 B1

Printed by Jouve, 75001 PARIS (FR)

## Description

The invention relates to a novel use of 8 substituted xanthine derivatives for the manufacture of a medicament for the treatment of auto-immuno disorders.

Methylxanthines, for example pentoxifylline (PTX) are known having immunosuppressive effects in vitro.

Several types of 8-substituted xanthine derivatives have been publicized, for example K.A. Jacobson et al. J. Med. Chem. 1993, 36, 2639-2644; K.A. Jacobson et al. Biochem. Pharmacol. 1988, 37, 3653-3661; K.A. Jacobson et al. J. Med. Chem. 1989, 32, 1873-1879.

Recently (Lin Y. et al, Transplantation 63 (1997) it has been found that the co-medication of an immunosuppressive compound such as cyclosporine A (CyA) or FK506 or RPM (rapamycine) with a methyl xanthine derivative, in particular A802715 (7-propyl-1(5-hydroxy-5-methylhexyl)-3-methylxanthine) leads to a superadditive increase in the immunosuppressive action.

The immunosuppressive effect of cyclosporine A (CyA) is already known since 1972. However, due to its nephrotoxicity and several other side effects CyA has not been able to establish itself as the optimal and final drug of choice.

The present invention relates to a novel use of 8-substituted xanthine derivates and their pharmaceutical salts, possessing unexpectedly desirable pharmaceutical properties, i.c. are immunosupressive agents.

The invention demonstrates a novel use of xanthine derivatives of the formula (I):

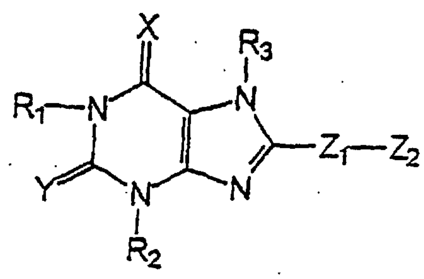

wherein:

$R_1$, $R_2$ and $R_3$ are independently hydrogen, saturated or unsaturated aliphatic chains which may be straight or branched having 1 to 6 carbon atoms;
X and Y are independently oxygen or sulfur;
$Z_1$ is selected from the group comprising a thie nyl; furanyl; cyclopentyl or a substituted by $Z_2$ or unsubstituted phenyl; wherein $Z_2$ is selected from the group comprising phenyl; sulfonic acid; unsubstituted or N-substituted sulfonamide with substituents such as alkyl, aminoalkyl where the amino group may be substituted itself with lower alkyl groups bearing 1 to 4 carbon atoms; nitro; halogen; substituted or unsubstituted amino group; aliphatic chain with 1 to 3 carbon atoms; halogenated aliphatic chain with 1 to 3 carbon atoms; aliphatic chain containing ether functions, acids, esters, amides, substituted or unsubstituted amines having 1 to 3 carbon atoms, nitro, sulfonamides or a combination of these functional groups with a maximum length of the chain of 12 atoms,

or a pharmaceutically acceptable salt thereof for the manufacture of a medicament for the treatment of auto-immune disorders.

The invention further relates to a combination preparation, containing 1) cyclosporin A or FK506 or rapamycin, or Leflunomide or Mofetil 2) at least one 8-substituted xanthine derivative of formula (I), and optionally a pharmaceutical excipient, for simultaneous, separate or sequential use in (auto)immune disorders.

The invention also relates to the use of the novel compounds of claims 6-10 as a medicament, and to the use of 1-propynyl-3,7-dimethylxantine (1) or a compound of claims 6-10 for the manufacture of a medicament for the treatment of auto-immune disorders.

Hereunder the effects of the 8-substituted xanthine derivatives on the Lymphocyte activation are elucidated and are compared with non-substituted xanthine derivatives (see table I, compound 1,2,3,4,5,22,23,24,25,26, and 67,68,69,70,71).

Table I summarizes the tested compounds. These xanthine.derivatives were obtained as follows:

**Compound number 8, 10, 12, 14, 21, 36, 37, 38, 47, 48, 50, 51, 79, 83**

K.A. Jacobson et al. J. Med. Chem. **1993,** 36, 2639-2644;

**9, 11, 13, 30, 31, 39, 40, 41, 42, 43, 125**
K.A. Jacobson et al. Biochem. Pharmacol. **1988,** 37, 3653-3661;

**15, 17, 18, 28, 29, 33, 34, 35, 44, 45, 46, 55, 56, 57, 58, 59, 60, 61, 62, 63, 64, 65, 78, 80, 81**
K.A. Jacobson et al. J. Med. Chem. 1989, 32, 1873-1879;

Compoundnumbers **1, 2, 3, 4, 5, 22, 23, 24, 67, 68, 82, 112, 113, 114, 116, 117, 118, 119** (table I and II) were obtained by the following procedure for the alkylation of xanthine derivatives.

0,01 mol of a xanthine derivative (**1a, 2a, 3a, 4a, 5a, 67a, 68a, 82a, 112a, 113a (114a), 116a (117a), 118a (119a)**). [The origin of these compounds is as follows: **1a** Theobromin, commercially available FLUKA AG; **2a** W. Traube, Ber. Deut. Chem. Ges. **33,** 3035 (1900); **3a** G. Elion, J. Org. Chem. **27,** 2478 (1962); **4a** W. Hutzenlaub, W. Pfleiderer, Liebigs Ann. Chem. **1979,** 1847; **5a** Xanthin, commercially available FLUKA AG; **67a** W. Hutzenlaub, W. Pfleiderer, Liebigs Ann. Chem. **1979**, 1847; **68a** P.G. Kjellin, C.G.A. Persson, Eur. Pat. Appl. 10 531; C.A. 94, P 15773 u; **82a** K.A. Jacobson, D. Shi, C. Gallo-Rodriguez, M. Manning, C. Müller, J.W. Daly, J.L. Neumeyer, L. Kiriasis, W. Pleiderer, J. Med. Chem. **36,** 2639 (1993); **112a** H. Goldner, G. Dietz, E. Carstens, Liebigs Ann. Chem. **691,** 142 (1966); **113a** M.T. Shamim, D. Ukena, W.L. Padgett, J.W. Daly, J. Med. Chem. **32,** 1231 (1989)] (see table II) were suspended or dissolved in DMF (60 ml) at room temperature and then under stirring $K_2CO_3$ (6 g per N-H function) and the alkylating agent (methyl iodide, ally iodide, propargyl bromide, n-propyl iodide, benzyl bromide, 2-chlorobenzyl bromide, 4-bromobutanoic acid, 5-bromopentanoic acid, ethyl 4-bromobutanoate) (0.015 mol per N-H) function added. The mixture was stirred at room temperature for 15 h, then the insoluble inorganic salts filtered off by suction and the filtrate evaporated in vacuum at 50°C to a syrup. The residue was treated with $H_2O$ forming a colorless solid. The precipitate was collected and purified by recrystallization from $H_2O$/EtOH mixtures to give colorless crystals of **1, 2, 3, 4, 5, 67, 68, 82, 112, 113, 114, 116, 117, 118, 119.**

Compound numbers **16**, **52, 53, 54, 69, 70, 71, 115, 116a (117a), 118a (119a)** (table I and III) were obtained by a procedure for the cyclization of 5-acylamino-6-aminouracils

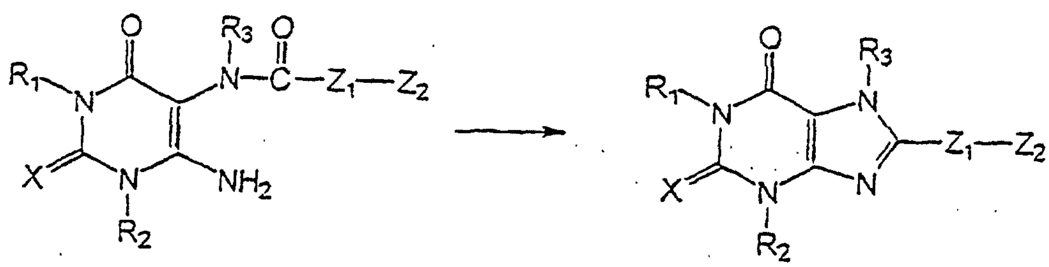

The 5-acylamino-6-aminouracil (**16a, 52a, 53a, 54a, 69a, 70a, 71a, 115a, 116b, 118b)** (0.01 mol) was heated in a mixture of 2 NaOH (50 ml) and EtOH (10 ml) under reflux for 30 min. The hot solution was acidified by AcOH whereby a colorless precipitate separated-. The solid was collected after cooling, dried and then purified by recrystallization from EtOH, DMF or by reprecipitation from alkaline solution by addition of AcOH.

**General procedures for the synthesis of 5-acylamino-6-amino-uracils (16a, 52a, 53a, 54a, 69a, 70a, 71a, 115a, 116b, 118b) (table IV).**

a) 0.01 mol of the N-substituted 5,6-diaminouracil **(69b, 70b, 71b)** was heated in formic acid (20 ml) for 15 mm under reflux. The reaction mixture was evaporated to dryness and the residue recrystallized from water to give colorless crystals **(69a, 70a, 71a).** Yield: 75-90%.

b) 0.01 mol of the N-substituted 5,6-diaminouracil (**52b, 53b, 116c**) was treated with 0.012 mol of the appropiate acyl chloride (p-nitrobenzoyl chloride, p-biphenyl-4-carbonyl chloride, p-chlorbenzoyl chloride, p-aminobenzoyl chloride) in abs. pyridine (20 ml) with stirring at room temp. for 3 hours. It was evaporated, the residue treated with water and the resulting precipitate collected by suction. Recrystallization from EtOH/$H_2O$ yielded 70-90% of colorless crystals (**52a, 115a, 53a, 54a, 116b, 118b**).

c) 0.01 mol of the N-substituted 5,6-diaminouracil (**16b**) was suspended in EtOH (100 ml), then subsequently added 0.011 mol of the appropiate acid (p-sulfamoylbenzoic acid) and 0.012 mol of the condensing agent (dicyclohexylcarbodiimide), N-dimethylaminopropyl-N'-ethylcarbodiimide hydrochloride). The mixture stirred at room temp. for 2 hours, the precipitate filtered off and purified by recrystallization from EtOH to give colorless crystals (**16a**). Yield: 80-90%.

**General procedure for the synthesis of N-alkyl-5,6-diaminouracil (16b, 52b, 53b, 69b, 70b, 71b, 116c) (table V).**

a) 0.05 mol of 6-amino-3-methyl-1-neopentyluracil (**69c**), 6-amino-1,3-dimethyluracil (**52c**), 6-amino-1,3-di-n-propyluracil (**53c**), 6-amino-1-isopropyl-3-methyluracil (**71c**), 6-amino-1-n-propyluracil (**116d**) and 6-amino-1,3-di-n-propyl-2-thiouracil (**16c**), respectively, were suspended in a mixture of water (100 ml) and EtOH (20 ml) and heated to 50°C. Then $NaNO_2$ (4 g) was added and the stirring mixture acidified by dropwise addition of AcOH (5 ml) whereby intermediary solution with strong coloration takes place. A red to violet coloured precipitate consisting of the corresponding 5-nitroso derivative was formed. The solid was collected after cooling (85-90%) and used directly for reduction to the anticipated N-alkyl-5,6-diaminouracil (**69b, 52b, 53b, 71b, 116c, 16b**). 0.05 mol of the 6-amino-N-alkyl-5-nitrosouracil derivative was added under stirring to a warm solution (50°C) of ammonium sulfide (25 ml) and then the temperature raised to 80°C for 15 min. On cooling the resulting precipitate was filtered off by suction, washed with water and little MeOH and then dried in a vacuum desiccator to give 75-90% of colorless to yellowish crystals.

b) **6-Amino-5-methylamino-1-neopentyluracil** (**70b**). 6-Amino-1-neopentyluracil (**70c**) (3.94 g, 0.02 mol ) was treated in AcOH (40 ml) at 80°C in presence of NaOAc x 3 $H_2O$ (2.6 g, 0.02 mol) with bromine (3.2 g, 0.02 mol) by dropwise addition. After 2 hours was cooled, the precipitate (75%) collected, washed with water and dried. 6-Amino-5-bromo-1-neopentyluracil (2.76 g, 0.01 mol) was then stirred in a 40% aqueous methylamine solution (80 ml) at room temp. for 2 days. The mixture was evaporated to half its volume and the precipitate collected. Washing with water and drying in a desiccator yielded 2.05 g (90%) of **70b**. M.P. 217-220°C.

**Syntheses of N-alkyl-6-aminouracils.**

**6-Amino-1-neopentyluracil (70c).** N-neopentylurea (13.0 g, 0.1 mol) and ethyl cyanoacetate (10 ml) were heated in 4N NaOEt (100 ml) for 4 hours under reflux. The reaction mixture was evaporated to dryness, the residue treated with water (100 ml) and then acidified with AcOH to pH 4-5 to form a colorless precipitate. Yield: 11.2 g (59%).

**6-Amino-3-methyl-1-neopentyluracil (69c).** 6-Amino-1-neopentyluracil (90c) (5.9 g, 0.03 mol) were dissolved in 1 N NaOH (50 ml) and then unrder vigorous stirring dimethylsulfate (3.9 ml, 0.033 mol) dropwise added at room temp. A precipitate separated and was collected after 2 hours. After washing with water and drying in a vacuum desiccator resulted 5.76 g (91%) of colorless crystals.

**6-Amino-3-methyl-1-n-isopropyluracil (71c).** 6-Amino-1-isopropyluracil (16.9 g, 0.1 mol) were dissolved in 1 N NaOH (120 ml) and then at room temp. dimethylsulfate (12 ml, 0.12 mol) dropwise added with vigorous stirring. After 1 hour the precipitate was collected, washed with water and dried at 50°C in high vacuum to give 15.1 g (82%) of chromatographically pure, colorless crystals.

**6-Amino-1-n-propyluracil (116d).** N-n-propylurea (20.4 g, 0.2 mol) and ethyl cyanoacetate (20 ml) were heated in 3 N NaOMe (200 ml) for 3 hours under reflux. The reaction mixture was evaporated, the residue treated with 100 ml of water and acidified with AcOH to pH 4 to give 23.7 g (70%) of colorless crystals.

**6-Amino-1,3-di-n-propyl-2-thiouracil (16c).** To a mixture of cyanoacetic acid (10 g) and acetic anhydride (50 ml) was added N,N'-di-n-propylthiourea (16 g, 0.01 mol) and stirred at 60°C for 4 hours. It was evaporated to dryness, the residue treated with 30% NaOH (100 ml) for 30 min, then diluted with water (100 ml) and the precipitate collected. Recrystallization from EtOH/water gave 18 g (79%) of yellowish crystals. The same procedure and started from N,N'-dimethylurea or N,N'-di-n-propylurea yielded 6-amino-1,3-dimethyluracil (**52c**) and 6-amino-1,3-di-n-propyluracil (**53c**) respectively as colorless crystals.

Compoundnumbers 25 and 26 (table I and table III) were obtained by a general procedure for the sythesis of 1-(5-hydroxyhexyl)xanthines.

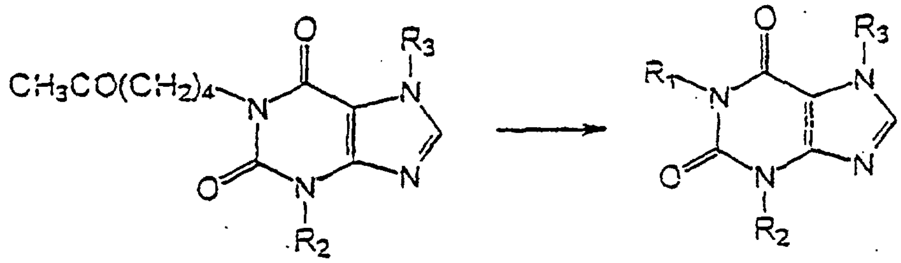

3,7-Dialkyl-1-(hexan-5-onyl)xanthine (2 mmol) was dissolved in MeOH (15 ml) and then treated under stirring with $NaBH_4$ (0.1 g) overnight. The mixture was evaporated to dryness, the residue diluted with $H_2O$, then extracted several times with $CHCl_3$. The $CHCl_3$ layer was dried over $Na_2SO_4$, filtered and the filtrate again evaporated to give a chromatographically pure solid. The solid was stirred in n-hexane for 1 h, then filtered by suction and dried in a vacuum destillator to give a colorless crystal powder.

Compounds **19, 20** and **66** were obtained by methylation of **55, 57** and **64** respectively. 0.01 Mol of the purine **55, 57** and **64**, respectively, was dissolved in DMF (120 ml) by warming. After cooling to room temperature $K_2CO_3$ (7 g) and methyl iodide (2 ml) were added and then the mixture stirred for 3 hours. The solution was then diluted with $H_2O$ (150 ml) and after cooling the precipitate collected, washed with water and dried. Recrystallization from EtOH/$H_2O$ gave colorless crystals in 75-90% yield. M.p. 198°C (**19**), 298°C (**20**) and 197°C (**66**).

The synthesis of compound **32** is based on compound 33, which is described in literature [K.A. Jacobson, K. L. Kirk, W.L. Padgett, J.W. Daly, J. Med. Chem. **1985**, 28, 1334]. Compound **33** (2.14 g, 0.005 mol) was suspended in abs. pyridine (50 ml) and then under stirring chlorosulfonic acid (4 ml) added dropwise. It was heated to 50°C with stirring for 12 hours. The reaction mixture was evaporated in vacuum, coevaporated twice with EtOH and the residue recrystallized from $H_2O$/EtOH to give 1.95 g (77%) of **32** of a colorless crystal powder. M.p. 245°c.

**Materials and methods**

Various models may be used for testing an immunosuppressive effect. In vivo, for example, different transplantation models are available. They are strongly influenced by different immunogenicities, depending on the donor and recipient species used and depending on the nature of the transplanted organ. The survival time of transplanted organs can thus be used to measure the suppression of the immune response. In vitro, there exist also various models. The most used are lymphocyte activation tests. Usually activation is measured via lymphocyte proliferation. Inhibition of proliferation thus always means immunosuppression under the experimental conditions applied. There exist different stimuli for lymphocyte activation:

- coculture of lymphocytes of different species (MLR = mixed lymphocyte reaction): lymphocytes expressing different minor and major antigens of the HLA-DR type (= allogens) activate each other non-specifically.
- CD3 assay: here there is an activation of the T-lymphocytes via an exogenously added antibody (OKT3). This antibody reacts against the CD3 molecule located on the lymphocyte membrane. This molecule has a costimulatory function. The interaction anti-CD3 (= OKT3)-CD3 results in T-cell activation which proceeds via the $Ca^{2}$+/calmodulin/cacineurin system and can be inhibited by CyA.
- CD28 assay: here specific activation of the T-lymphocyte goes also via an exogenously added antibody against the CD28 molecule. This molecule is also located on the lymphocyte membrane, and delivers strong costimulatory signals. This activation is $Ca^{2}$+-independent and thus cannot be inhibited by CyA.
- IL-2R assay: here activation of the lymphocyte occurs via the exogenously added cytokine IL-2 which binds to the IL-2 receptor (IL-2R) that is located on the lymphocyte membrane of prestimulated T cells. This activation is also $Ca^{2}$+/cAMP-independent and cannot be inhibited by CyA.

Reagents

All derivatives were dissolved in 0.5 ml DMSO and further diluted in culture medium before use in in vitro experiments. The culture medium consisted of RPMI-1640 + 10% FCS.

Mixed Lymphocyte Reaction

Peripheral blood mononuclear cells (PBMC) were isolated from heparinized peripheral blood by density gradient centrifugation over Lymphoprep (Nycomed, Maorstua, Norway). Allogeneic PBMC or EBV-transformed human B cells [RPMI1788 (ATCC name CCL156)] which strongly express B7-1 and B7-2 were used as stimulator cells after irradiation with 30 Gy. MLR was'performed in triplicate wells. After 5 days incubation at 37°C, 1 μCi [$^3$H]-thymidine was added to each cup. After a further 16 hours incubation, cells were harvested and counted in a β-counter.

The percent suppression of proliferation by drugs was counted using the formula:

$$\text{Per cent inhibition} = \frac{\text{(cpm+drugs)-cpm Cult.Med.)}}{\text{(cpm-drugs)-cpm Cult. Med.)}} \times 100$$

T cell purification

T cells were purified by removing non-T cells. Briefly, monocytes were removed by cold agglutination. The resulting lymphoid cells were further purified by a cell enrichment immunocolumn [Cellect Human T (Biotex, Edmonton, Alberta, Canada)] by a process of negative selection. More than 95% of the B cells were removed with this procedure. After depletion, the resulting T cell preparation was highly purified explaining these cells could not be activated by PHA or rIL-2 alone at concentrations capable of stimulating RBMC prior to deletion.

Measurements of T cell proliferations induced by anti-CD3 mAb + PMA or anti-CD28 mAb + PMA

Highly purified T cells ($10^6$/ml) were stimulated by immobilized anti-CD3 or anti-CD28 mAb in the presence of PMA. Anti-CD3 mAb (CLB-CD3; CLB, Amsterdam, The Netherlands) were fixed on the 96-microwell plates by incubating the wells with 50 μl of mAb solution (CLB-CD28; CLB, Amsterdam, The Netherlands) 50 μl (1/650 dilution in culture medium) was added directly to the wells. Further, 20 μl PMA (Sigma, St. Louis, MO, USA) solution (final concentration: 0.5 ng/ml) was added. Subsequently, 20 μl of immunosuppressants were added by serial dilution in triplicate wells. Finally 100 μl of the T cell suspension ($10^6$/ml) was added. After 48-hour incubation at 37°C in 5% $CO_2$ 20 μl BrdU (100 μM solution) (Cell Proliferation Elisa, Boehringer-Mannheim Belgium) was added to each well. After a further overnight incubation the T cell proliferation was measured using a colorimetric immunoassay for qualification of cell proliferation based on measurements of the incorporation of BrdU during DNA synthesis. The optical density (OD) was measured by a Behring EL311 place reader at 450 nm (reference wavelength: 690 nm). The percent suppression of proliferation by drugs was counted using the formula:

$$\text{Per cent inhibition} = \frac{\text{(OD+drugs)-(OD Cult. Med.)}}{\text{(OD-drugs)-(OD Cult. Med.)}} \times 100$$

In_vitro immunosuppressive effect of Xanthine Derivatives as measured with the MLR and with tests involving polyclonal T cell proliferation induced by anti-CD3 mAb + PMA or anti-CD28 mAb + PMA (table VI)

- In the tabe VI column II shows the IC50 values of the various substances in the MLR. The IC50 value represents the lowest concentration of the substances that resulted in a 50% suppression of the MLR.
- Column III shows the IC50 value of the various substances for the anti-CD3 mAb + PMA pathway and row IV the IC50 values of the various substances for the anti-CD28 mAb + PMA pathway.
- As a comparison the values of other immunosuppressants: CsA, FK506, Rapamycin, Leflunomide and Mycophenolic acid are given as well.

Whole Blood Assay (WBA): WBA is a lymphoproliferation assay performed in vitro but using lymphocytes present in whole blood, taken from animals that were previously given test substances in vivo. Hence it reflects the in vivo effect of substances as assessed with an in vitro read-out assay.

Rats: inbred, male 6- to 8-weeks old R/A rats weighing ± 200 g were used as recipients.

Drug administration: Xanthine derivatives were dissolved in DMSO and further diluted with PBS. Products were given orally in different concentrations 2 times a day for 2 days. To perform the experiments, 6-8 hours after the last administration 1 ml of blood is taken by heart puncture after ether anesthesia and anticoagulated with 100 U/ml of preservative free heparine.

Whole Blood Assay: This assay was performed as we described previously [Use of the Methylxanthine Derivatives A802715 in Transplantation Immunology. II In vitro Experiments. (Yuan Lin, et al., Transplantation 1997, 63, No. 12, 1734-1738)].

Heparinized whole blood was diluted (1:25) with complete RPMI medium and stimulated with 15 μg/ml of

concanavalin A (Con A) in triplicate wells in 96-well microtiter plates at 37°C and 5% $CO_2$. After 96-h culture, proliferation was determined by measuring the incorporation (cpm) of [$^3$H]-thymidine.

The Con A induced proliferation of lymphocytes taken from rats receiving the test substances (exp) was compared with that from rats receiving only the solvent (con). The percent suppression was calculated as follows:

$$\%\ \text{suppression} : 100 - \left[ \frac{\text{cpm exp}}{\text{cpm con}} \times 100 \right]$$

| Results | | | |
|---|---|---|---|
| Nr | % suppression | Administration of drugs | Blood taken after; |
| 11 | 33 | 40mg/kg/d 2x/d 2d | 8 h |
| 14 | 86 | 40mg/kg/d 2x/d 2d | 8 h |

First, most of the substances according to the invention have a clear suppressive effect in the MLR (mixed lymphocyte reaction). The MLR is considered as an in vitro analogue of the transplant rejection as it is based on the recognition of allogeneic MHC (major histocompatibility antigens) on the stimulator leucotyes, by responding lymphocytes. Various established immunosuppressive drugs are known to suppress the MLR, and were also shown in this description. Further, the 8-substitutd xanthine derivatives are more effective than the non-substituted.

From these data it can be deduced that the 8-substituted xanthine derivatives may be effective in clinical situations where other immunosuppressants are active as well.

These include the prevention and/or treatment of organ transplant rejection, the prevention and/or treatment of both rejection and the occurrence of graft-versus-host-disease after BM transplantation; the prevention and/or treatment of autoimmune diseases including diabetes mellitus, multiple sclerosis, glomerulonephritis, rheumatoid arthritis, proriasis systemic diseases such as vasculitis; scleroderma, polymyositis, autoimmune endocrine disorders (thyroiditis), ocular diseases (uveitis), inflammatory bowel diseases (Crohn's disease, colitis uclerosa), autoimmune liver diseases (autoimmune hepatitis, primary biliary cirrhosis) autoimmune pneumonitis and auto-immune carditis.

Whereas cyclosporine A and FK506 are only active in the anti-CD3 + PMA test, the 8-substituted xanthine derivatives according to the invention were active, not only in the anti-CD3 + PMA but also in the anti-CD28 + PMA test. It has been shown that the latter is Ca-calmodulin resistant, and resistant to CsA and FK506. The anti-CD28 + PMA pathway has also been called the cosignal pathway and is important to induce energy and even tolerance in T cells. Moreover, representative compounds have been found to be active in a whole blood assay.

Under the term "organ" in the description is understood all organs or parts of organs (even several) in mammals, in particular humans, for example kidney, heart, skin, liver, muscle, cornea, bone, bone marrow, lung, pancreas, intestine or stomach.

After organ transplantation, rejection of the transplanted organ by the recipient occurs (host-versus-graft reaction). After bone marrow transplantation, also rejection of the host by the grafted cell may occur (graft-versus-host reaction). Rejection reactions mean all reactions of the recipient body or of the transplanted organ which in the end lead to cell or tissue death in the transplanted organ or adversely affect the functional ability and viability of the transplanted organ or adversely affect the functional ability and viability of the transplanted organ or the recipient. In particular, this means acute and chronic rejection reactions.

Auto-immune disorders include, inter alia, systemic lupus erythematosus, rheumatoid arthritis, psoriasis, pemphigus, atopic dermatitis, myositis, multiple sclerosis, nephrotic syndrome (in particular glomerulonephritis), ulcerative colitis or juvenile diabetes.

The invention further relates tp the use of cyclosporin A or FK506 or Rapamycine or Leflunomide or Mofetil and at least one 8-substituted xanthine according to the invention for the production of a pharmaceutical for inhibiting the replication of viruses such as picorna-, toga-, bunya-, orthomyxo-, paramyxo-, rhabdo-, retro-, arena-, hepatitis B-, hepatitis C-, hepatitis D-, adeno-, vaccinia-, papilloma-, herpes-, varicella-zoster-virus or human immunodeficiency virus (HIV); or for treating of cancer such as lung cancers, leukaemia, ovarian cancers, sarcoma, Kaposi's sarcoma, meningioma, colon cancers, lymp node tumors, glioblastoma multiforme, prostate cancers or skin carcinoses.

The invention further relates to the use of cyclosporin A or FK506 or rapamycin or Leflunomide or Mofetil and at least one xanthine of the general formula for the production of a pharmaceutical for the treatment of human after organ transplantation or of (auto)immune disorders.

Hence, the advantage to associate xanthine with other immunosuppressants may be that, first, the therapeutic spectrum of action of the individual components is quantitatively and qualitatively broadened. Secondly that it allows, by means of a dose reduction without reduced efficacy but with increased safety, that the treatment of immune disorders which were hitherto no indication for immunosuppressive therapy as a result of side effects may be considered. At the same time, the therapy costs can be decreased to an appreciable extent.

The prefered compounds according to the invention are the xantine derivates bearing on the 8-position a substituted or unsubstituted phenyl.

Table 1

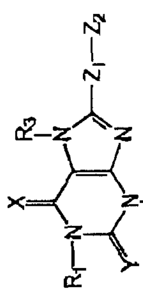

| Compound n° | $R_1$ | $R_1$ | $R_3$ | X | Y | $Z_1$ | $Z_2$ |
|---|---|---|---|---|---|---|---|
| 1 | $CH_2C{\equiv}CH$ | $CH_3$ | $CH_3$ | O | O | H | - |
| 2 | $CH_2C{\equiv}CH$ | $CH_3$ | $CH_2C{\equiv}CH$ | O | O | H | - |
| 3 | $CH_3$ | $CH_2C{\equiv}CH$ | $CH_2C{\equiv}CH$ | O | O | H | - |
| 4 | $CH_2C{\equiv}CH$ | $CH_2C{\equiv}CH$ | $CH_3$ | O | O | H | - |
| 5 | $CH_2C{\equiv}CH$ | $CH_2C{\equiv}CH$ | $CH_2C{\equiv}CH$ | O | O | H | - |
| 8 | $CH_2$-$CH$=$CH_2$ | $CH_3$ | $CH_2$-$CH$=$CH_2$ | O | O | 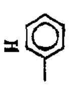 | - |
| 9 | $CH_2CH_2CH_3$ | $CH_2CH_2CH_3$ | H | O | O | 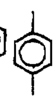 | -$SO_2NHCH_2CH_2NMe_2$ |
| 10 | $CH_3$ | $CH_3$ | $CH_3$ | O | O | 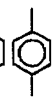 | $CF_3$ |
| 11 | $CH_2CH_2CH_3$ | $CH_2CH_2CH_3$ | H | O | O | 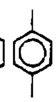 | -$SO_2NH(CH_2)_3NEt_2$ |
| 12 | $CH_2$-$CH$=$CH_2$ | $CH_3$ | $CH_3$ | O | O | 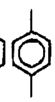 | $CF_3$ |
| 13 | $CH_2CH_2CH_3$ | $CH_2CH_2CH_3$ | H | O | O | 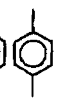 | -$OCH_2CONH(CH_2)_2NEt_2$ |
| 14 | $CH_2CH_2CH_3$ | $CH_2CH_2CH_3$ | $CH_2CH$=$CH_2$ | O | O | 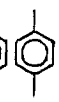 | $CF_3$ |
| 15 | $CH_2CH_2CH_3$ | $CH_2CH_2CH_3$ | H | O | O | 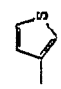 | - |

| Compound n° | $R_1$ | $R_2$ | $R_3$ | X | Y | $Z_1$ | $Z_2$ |
|---|---|---|---|---|---|---|---|
| 16 | $CH_2CH_2CH_3$ | $CH_2CH_2CH_3$ | H | O | S | 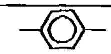 | $SO_2NH_2$ |
| 17 | $CH_3$ | $CH_3$ | H | S | O | 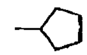 | - |
| 18 | $CH_3$ | $CH_3$ | H | S | S | 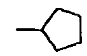 | - |
| 19 | $CH_3$ | $CH_3$ | $CH_3$ | O | O | 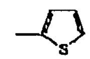 | - |
| 20 | $CH_3$ | $CH_3$ | $CH_3$ | O | S | 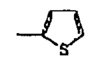 | - |
| 21 | $CH_2CH=CH_2$ | $CH_3$ | $CH_3$ | O | O | 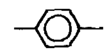 | $SO_3Na$ |
| 22 | $-(CH_2)_4COOH$ | $CH_3$ | $CH_3$ | O | O | H | - |
| 23 | $-(CH_2)_3COOH$ | $CH_3$ | $CH_3$ | O | O | H | - |
| 24 | $-(CH_2)_3COOEt$ | $CH_3$ | $CH_3$ | O | O | H | - |
| 25 | $-(CH_2)_4CHOH-CH_3$ | $CH_3$ | $CH_3$ | O | O | H | - |
| 26 | $-(CH_2)_4CHOH-CH_3$ | $CH_3$ | $CH_2CH_2CH_3$ | O | O | H | - |
| 28 | $-CH_2CH_2CH_3$ | $CH_2CH_2CH_3$ | H | O | S | 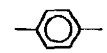 | $-OCH_2COOH$ |
| 29 | $-CH_2CH_2CH_3$ | $CH_2CH_2CH_3$ | H | O | S | 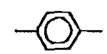 | $-OCH_2COOEt$ |
| 30 | $CH_2CH_2CH_3$ | $CH_2CH_2CH_3$ | H | O | O | 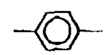 | $OCH_2COOMe$ |
| 31 | $CH_2CH_2CH_3$ | $CH_2CH_2CH_3$ | H | O | O | 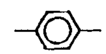 | $OCH_2CONH(CH_2)_2NHCOCH_3$ |
| 32 | $CH_2CH_2CH_3$ | $CH_2CH_2CH_3$ | H | O | O | 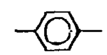 | $OCH_2CONH(CH_2)_2NHSO_3H$ |

| Compound n° | $R_1$ | $R_2$ | $R_3$ | X | Y | $Z_1$ | $Z_2$ |
|---|---|---|---|---|---|---|---|
| 33 | $CH_2CH_2CH_3$ | $CH_2CH_2CH_3$ | H | O | O | 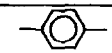 | $OCH_2CONH(CH_2)_2$-$NH_2$ |
| 34 | $CH_2CH_2CH_3$ | $CH_2CH_2CH_3$ | H | O | O | 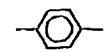 | $OCH_2CONH(CH_2)_2NMe_2$ |
| 35 | $CH_2CH_2CH_3$ | $CH_2CH_2CH_3$ | H | O | S | 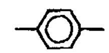 | $OCH_2CONH(CH_2)_2NH_2$ |
| 36 | $CH_2CH_2CH_3$ | $CH_2CH_2CH_3$ | H | O | O | 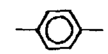 | -$SO_2NH_2$ |
| 37 | H | $CH_3$ | $CH_3$ | O | O | 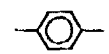 | $SO_2NH_2$ |
| 38 | H | $CH_3$ | $CH_3$ | O | O | 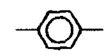 | $SO_3H$ |
| 39 | $CH_3$ | $CH_3$ | H | O | O | 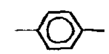 | $SO_2NH(CH_2)_3NH_2$ |
| 40 | $CH_2CH_2CH_3$ | $CH_2CH_2CH_3$ | H | O | O | 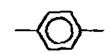 | $SO_2NH(CH_2)_3NMe_2$ |
| 41 | $CH_3$ | $CH_3$ | H | O | O | 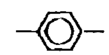 | $SO_2NH(CH_2)_2NMe_2$ |
| 42 | $CH_3$ | $CH_3$ | H | O | O | 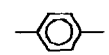 | $SO_2NH(CH_2)_3NMe_2$ |
| 43 | $CH_3$ | $CH_3$ | H | O | O | 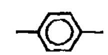 | $SO_2NH(CH_2)_3NEt_2$ |
| 44 | $CH_3$ | $CH_3$ | H | O | S | 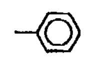 | - |
| 45 | $CH_3$ | $CH_3$ | H | S | O | 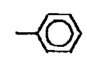 | - |
| 46 | $CH_2CH_3$ | $CH_2CH_3$ | H | S | O | 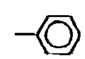 | - |

| Compound n° | R | $R_1$ | $R_2$ | X | Y | $Z_1$ | $Z_2$ |
|---|---|---|---|---|---|---|---|
| 47 | H | $CH_3$ | $CH_3$ | O | O | 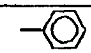 | - |
| 48 | $CH_2CH_2CH_3$ | $CH_2CH_2CH_3$ | H | O | O | 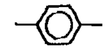 | $CF_3$ |
| 50 | $CH_2CH{=}CH_2$ | $CH_3$ | $CH_3$ | O | O | 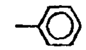 | - |
| 51 | $CH_3$ | $CH_3$ | H | O | O | 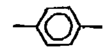 | $CF_3$ |
| 52 | $CH_3$ | $CH_3$ | H | O | O | 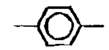 | $NO_2$ |
| 53 | $CH_2CH_2CH_3$ | $CH_2CH_2CH_3$ | H | O | O | 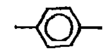 | $NO_2$ |
| 54 | $CH_2CH_2CH_3$ | $CH_2CH_2CH_3$ | H | O | O | 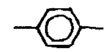 | $NH_2$ |
| 55 | $CH_3$ | $CH_3$ | H | O | O | 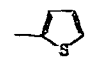 | - |
| 56 | $CH_2CH_2CH_3$ | $CH_2CH_2CH_3$ | H | O | O | 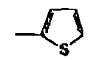 | - |
| 57 | $CH_3$ | $CH_3$ | H | O | S | 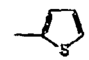 | - |
| 58 | $CH_3$ | $CH_3$ | H | O | O | 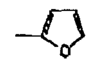 | - |
| 59 | $CH_2CH_2CH_3$ | $CH_2CH_2CH_3$ | H | O | O | 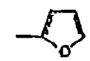 | - |
| 60 | $CH_3$ | $CH_3$ | H | O | S | 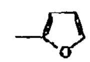 | - |

| Compound n° | $R_1$ | $R_2$ | $R_3$ | X | Y | $Z_1$ | $Z_2$ |
|---|---|---|---|---|---|---|---|
| 61 | $CH_2CH_2CH_3$ | $CH_2CH_2CH_3$ | H | O | S | 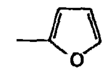 | - |
| 62 | $CH_3$ | $CH_3$ | H | O | O | 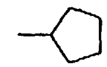 | - |
| 63 | $CH_2CH_2CH_3$ | $CH_2CH_2CH_3$ | H | O | O | 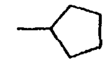 | - |
| 64 | $CH_3$ | $CH_3$ | H | O | S | 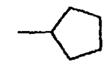 | - |
| 65 | $CH_2CH_2CH_3$ | $CH_2CH_2CH_3$ | H | O | S | 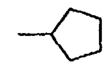 | - |
| 66 | $CH_3$ | $CH_3$ | $CH_3$ | O | S | 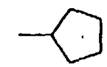 | - |
| 67 | $CH_3$ | $CH_2C{\equiv}CH$ | $CH_3$ | O | O | H | - |
| 68 | $CH_3$ | $CH_2C(CH_3)_3$ | $CH_3$ | O | O | H | - |
| 69 | $CH_3$ | $CH_2\text{-}C(CH_3)_3$ | H | O | O | H | - |
| 70 | H | $CH_2C(CH_3)_3$ | $CH_3$ | O | O | H | - |
| 71 | $CH_3$ | $-CH(CH_3)_2$ | H | O | O | H | - |
| 78 | $CH_3$ | $CH_3$ | H | O | O | 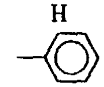 | - |
| 79 | $CH_3$ | $CH_3$ | $CH_3$ | O | O | 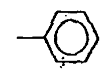 | - |
| 80 | $CH_2CH_2CH_3$ | $CH_2CH_2CH_3$ | H | O | O | 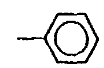 | - |
| 81 | $CH_2CH_2CH_3$ | $CH_2CH_2CH_3$ | H | O | S | 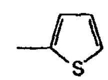 | |
| 82 | $CH_3$ | $CH_3$ | $CH_2CH{=}CH_2$ | O | O | 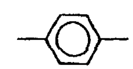 | $CF_3$ |

| Compound n° | $R_1$ | $R_2$ | $R_3$ | X | Y | $Z_1$ | $Z_2$ |
|---|---|---|---|---|---|---|---|
| **83** | H | $CH_3$ | $CH_3$ | O | O | 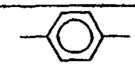 | $CF_3$ |
| **112** | $CH_3$ | $CH_3$ | $CH_2CH_2CH_3$ | O | O | 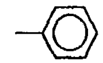 | - |
| **113** | $CH_2CH_2CH_3$ | $CH_2CH_2CH_3$ | $CH_2CH_2CH_3$ | O | O | 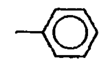 | - |
| **114** | $CH_2$-$CH$=$CH_2$ | -$CH_2CH_2CH_3$ | $CH_2$-$CH$=$CH_2$ | O | O | 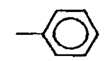 | - |
| **115** | $CH_3$ | $CH_3$ | H | O | O | 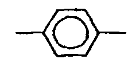 | 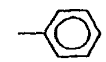 |
| **116** | $CH_2CH_2CH_3$ | $CH_2CH_2CH_3$ | $CH_2CH_2CH_3$ | O | O | 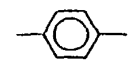 | 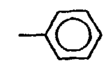 |
| **117** | $CH_2$-$CH$=$CH_2$ | $CH_2CH_2CH_3$ | $CH_2$-$CH$=$CH_2$ | O | O | 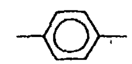 | 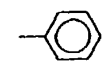 |
| **118** | $CH_2CH_2CH_3$ | $CH_2CH_2CH_3$ | $CH_2CH_2CH_3$ | O | O | 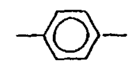 | Cl |
| **119** | $CH_2$-$CH$=$CH_2$ | $CH_2CH_2CH_3$ | $CH_2$-$CH$=$CH_2$ | O | O | 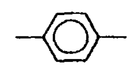 | Cl |
| **125** | $CH_3$ | $CH_3$ | H | O | O | 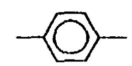 | -$SO_2NH_2$ |

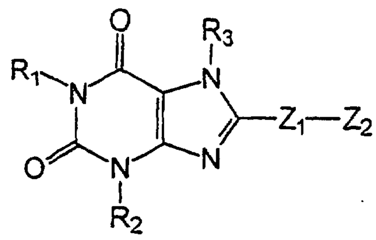

| | $R_1$ | $R_2$ | $R_3$ | $Z_1$ | $Z_2$ | Mp |
|---|---|---|---|---|---|---|
| 1a | H | $CH_3$ | $CH_3$ | H | - | |
| 1 | $HC{\equiv}C\text{-}CH_2$ | $CH_3$ | $CH_3$ | H | - | 204° |
| 2a | H | $CH_3$ | H | H | - | |
| 2 | $HC{\equiv}C\text{-}CH_2$ | $CH_3$ | $HC{\equiv}C\text{-}CH_2$ | H | - | 177° |
| 3a | $CH_3$ | H | H | H | - | |
| 3 | $CH_3$ | $HC{\equiv}C\text{-}CH_2$ | $HC{\equiv}C\text{-}CH_2$ | H | - | 174° |
| 4a | H | H | $CH_3$ | H | - | |
| 4 | $HC{\equiv}C\text{-}CH_2$ | $HC{\equiv}C\text{-}CH_2$ | $CH_3$ | H | - | 172° |
| 5a | H | H | H | H | - | |
| 5 | $HC{\equiv}C\text{-}CH_2$ | $HC{\equiv}C\text{-}CH_2$ | $HC{\equiv}C\text{-}CH_2$ | H | - | 155° |
| 22 | $HOOC(CH_2)_4$ | $CH_3$ | $CH_3$ | H | - | 195° |
| 23 | $HOOC(CH_2)_3$ | $CH_3$ | $CH_3$ | H | - | 208-210° |
| 24 | $EtOOC(CH_2)_3$ | $CH_3$ | $CH_3$ | H | - | 86-88° |
| 67a | $CH_3$ | H | $CH_3$ | H | - | |
| 67 | $CH_3$ | $HC{\equiv}C\text{-}CH_2$ | $CH_3$ | H | - | |
| 68a | H | $CH_2C(CH_3)_3$ | H | H | - | |
| 68 | $CH_3$ | $CH_2C(CH_3)_3$ | $CH_3$ | H | - | 158° |
| 82a | $CH_3$ | $CH_3$ | H | $-C_6H_4-$ | $p\text{-}CF_3$ | |
| 82 | $CH_3$ | $CH_3$ | $CH_2{=}CHCH_2$ | $-C_6H_4-$ | $p\text{-}CF_3$ | 116-118° |
| 112a | $CH_3$ | $CH_3$ | H | $-C_6H_5$ | - | |
| 112 | $CH_3$ | $CH_3$ | $CH_3CH_2CH_2$ | $-C_6H_5$ | - | 141° |
| 113a | H | $CH_3CH_2CH_2$ | H | $-C_6H_5$ | - | |
| 113 | $CH_3CH_2CH_2$ | $CH_3CH_2CH_2$ | $CH_3CH_2CH_2$ | $-C_6H_5$ | - | 123-125° |
| 114a | H | $CH_3CH_2CH_2$ | H | $-C_6H_5$ | - | |
| 114 | $CH_2{=}CHCH_2$ | $CH_3CH_2CH_2$ | $CH_2{=}CHCH_2$ | $-C_6H_5$ | - | 113-114° |
| 116a | H | $CH_3CH_2CH_2$ | H | $-C_6H_4$ | $p\text{-}C_6H_5$ | |
| 116 | $CH_3CH_2CH_2$ | $CH_3CH_2CH_2$ | $CH_3CH_2CH_2$ | $-C_6H_4$ | $p\text{-}C_6H_5$ | 116° |

| | $R_1$ | $R_2$ | $R_3$ | $Z_1$ | $Z_2$ | Mp |
|---|---|---|---|---|---|---|
| 117a | H | $CH_3CH_2CH_2$ | H | $-C_6H_4$ | p-$C_6H_5$ | |
| 117 | $CH_2{=}CHCH_2$ | $CH_3CH_2CH_2$ | $CH_2{=}CHCH_2$ | $-C_6H_4$ | p-$C_6H_5$ | 104-106° |
| 118a | H | $CH_3CH_2CH_2$ | H | $-C_6H_4$ | p-Cl | |
| 118 | $CH_3CH_2CH_2$ | $CH_3CH_2CH_2$ | $CH_3CH_2CH_2$ | $-C_6H_4$ | p-Cl | 71-74° |
| 119a | H | $CH_3CH_2CH_2$ | H | $-C_6H_4$ | p-Cl | |
| 119 | $CH_2{=}CHCH_2$ | $CH_3CH_2CH_2$ | $CH_2{=}CHCH_2$ | $-C_6H_4$ | p-Cl | 89-91° |

| | $R_1$ | $R_2$ | $R_3$ | X | $Z_1$ | $Z_2$ | Mp |
|---|---|---|---|---|---|---|---|
| 16 | $CH_3CH_2CH_2$ | $CH_3CH_2CH_2$ | H | S | $-C_6H_4$ | p-$SO_2NH_2$ | > 300° |
| 25 | $CH_3CHOH(CH_2)_4$ | $CH_3$ | $CH_3$ | | | | 118-120° |
| 26 | $CH_3CHOH(CH_2)_4$ | $CH_3$ | $CH_3CH_2CH_2$ | | | | 72-74° |
| 52 | $CH_3$ | $CH_3$ | H | O | $-C_6H_4$ | p-$NO_2$ | 275° |
| 53 | $CH_3CH_2CH_2$ | $CH_3CH_2CH_2$ | H | O | $-C_6H_4$ | p-$NO_2$ | > 270° |
| 54 | $CH_3CH_2CH_2$ | $CH_3CH_2CH_2$ | H | O | $-C_6H_4$ | p-$NO_2$ | > 300° |
| 69 | $CH_3$ | $CH_2C(CH_3)_3$ | H | O | H | - | 234° |
| 70 | H | $CH_2C(CH_3)_3$ | $CH_3$ | O | H | - | 248° |
| 71 | $CH_3$ | $CH(CH_3)_2$ | H | O | H | - | 230° |
| 115 | $CH_3$ | $CH_3$ | H | O | $-C_6H_4-$ | p-$C_6H_5$ | > 300° |

Table IV

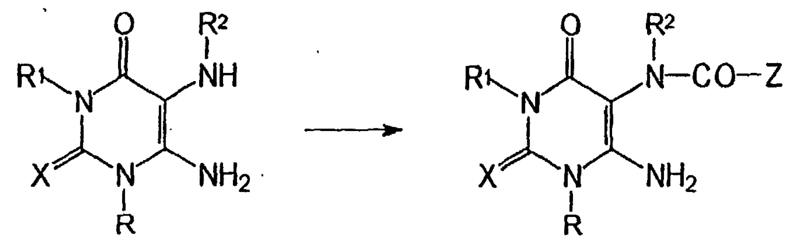

| | R | $R^1$ | $R^2$ | X | | R | $R^1$ | $R^2$ | X | Z | M.p. |
|---|---|---|---|---|---|---|---|---|---|---|---|
| 16b | n-Pr | n-Pr | H | S | 16a | n-Pr | n-Pr | H | S | p-$C_6H_4$-$SO_2NH_2$ | 268-270° |
| 52b | Me | Me | H | O | 52a | Me | Me | H | O | p-$C_6H_4$-$NO_2$ | |
| 53b | n-Pr | n-Pr | H | O | 53a | n-Pr | n-Pr | H | O | p-$C_6H_4$-$NO_2$ | >250° dec |
| 69b | $CH_2CMe_3$ | Me | H | O | 54a | n-Pr | n-Pr | H | O | p-$C_6H_4$-$NH_2$ | |
| 71b | $CHMe_2$ | Me | H | O | 69a | $CH_2CMe_3$ | Me | H | O | H | 235-237° |
| 70b | $CH_2CMe_3$ | H | Me | O | 71a | $CHMe_2$ | $CH_3$ | H | O | H | |
| 116c | n-Pr | H | H | O | 70a | $CH_2CMe_3$ | H | $CH_3$ | O | H | 267-270° |
| | | | | | 116b | n-Pr | H | H | O | 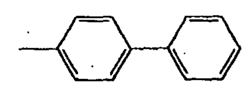 | 288-290° |
| | | | | | 115a | Me | Me | H | O | 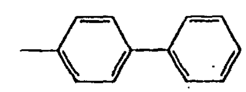 | 180° |
| | | | | | 118b | n-Pr | H | H | O | 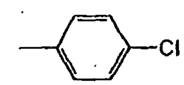 | 269-272° |

Table V

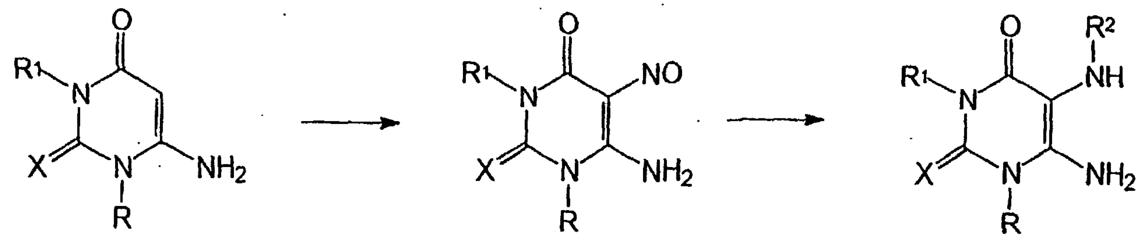

| | R | $R^1$ | X | | R | $R^1$ | $R^2$ | X | M.p. |
|---|---|---|---|---|---|---|---|---|---|
| 70c | $CH_2CMe_3$ | H | O | 70b | $CH_2CMe_3$ | H | Me | O | >237° dec |
| 69c | $CH_2CMe_3$ | Me | O | 69b | $CH_2CMe_3$ | Me | H | O | 108-110° |
| 16c | n-Pr | n-Pr | S | 16b | n-Pr | n-Pr | H | S | 110-113° |
| 52c | Me | Me | O | 52b | Me | Me | H | O | |
| 53c | n-Pr | n-Pr | O | 53b | n-Pr | n-Pr | H | O | |
| 71c | $CHMe_2$ | Me | O | 71b | $CHMe_2$ | Me | H | O | |
| 116d | n-Pr | H | O | 116c | n-Pr | H | H | O | |

| Nr | IC50 in μM | | | Nr | IC50 in μM | | |
|---|---|---|---|---|---|---|---|
| | Xanthine derivatives | | | | Xanthine derivatives | | |
| | MLR | aCD3 | aCD28 | | MLR | aCD3 | aCD28 |
| 1 | > 200 | 150 | > 200 | 25 | > 200 | 140 | 160 |
| 2 | > 200 | > 200 | 100 | 26 | 100 | 100 | 100 |
| 3 | 150 | 150 | 100 | 28 | 120 | 150 | 75 |
| 4 | > 200 | 90 | > 200 | 29 | > 200 | 150 | 130 |
| 5 | > 200 | 50 | > 200 | 30 | > 200 | 140 | 100 |
| 8 | 30 | 35 | 80 | 31 | 200 | 120 | 80 |
| 9 | 25 | 40 | 50 | 32 | 70 | 90 | 110 |
| 10 | 50 | 20 | 30 | 33 | 160 | 45 | 35 |
| 11 | 25 | 40 | 55 | 34 | 105 | 45 | 60 |
| 12 | 30 | 90 | 80 | 35 | 50 | 50 | 70 |
| 13 | ND | 35 | 40 | 36 | > 200 | 45 | 40 |
| 14 | 15 | 40 | 35 | 37 | > 200 | 150 | 150 |
| 15 | ND | > 200 | 170 | 38 | > 200 | 120 | 120 |
| 16 | ND | 25 | 20 | 39 | 100 | 120 | 140 |
| 17 | 80 | 30 | 40 | 40 | 25 | 60 | 70 |
| 18 | 120 | 75 | 40 | 41 | 120 | 80 | 90 |
| 19 | ND | 50 | 80 | 42 | 170 | 130 | 130 |
| 20 | ND | 170 | 50 | 43 | 115 | 120 | 90 |
| 21 | ND | 180 | 80 | 44 | 120 | 170 | 120 |
| 22 | > 200 | > 200 | > 200 | 45 | 165 | 25 | 25 |
| 23 | > 200 | > 200 | > 200 | 46 | > 200 | 25 | 20 |
| 24 | > 200 | 170 | 150 | | | | |

| Nr | IC50 in µM | | |
|---|---|---|---|
| Xanthine derivatives | | | |
| | MLR | aCD3 | aCD28 |
| 47 | 200 | 140 | 140 |
| 48 | 180 | 160 | 150 |
| 49 | ND | ND | ND |
| 50 | 180 | 200 | 120 |
| 51 | 200 | 200 | 200 |
| 52 | 80 | 180 | 90 |
| 53 | 110 | 160 | 110 |
| 54 | 120 | 130 | 130 |
| 55 | > 200 | 200 | 120 |
| 56 | > 200 | 170 | 100 |
| 57 | > 200 | > 200 | 180 |
| 58 | > 200 | 160 | 170 |
| 59 | 15 | 155 | 135 |
| 60 | > 200 | 200 | 190 |
| 61 | 100 | 170 | 110 |
| 62 | > 200 | > 200 | 190 |
| 63 | > 200 | 135 | 100 |
| 64 | > 200 | > 200 | > 200 |
| 65 | > 200 | 135 | 75 |
| 66 | > 200 | 170 | 170 |
| 67 | > 200 | > 200 | 200 |
| 68 | 75 | 130 | 120 |
| 69 | 120 | 110 | 45 |
| 70 | > 200 | 180 | 140 |
| 71 | 160 | | |

| Nr | IC50 in µM | | |
|---|---|---|---|
| Xanthine derivatives | | | |
| | MLR | aCD3 | aCD28 |
| 77 | 130 | | |
| 78 | > 200 | > 200 | > 200 |
| 79 | 75 | 100 | 130 |
| 80 | 160 | 120 | 65 |
| 81 | > 200 | 180 | 110 |
| 82 | 25 | 80 | 80 |
| 83 | > 200 | > 200 | 150 |
| 112 | 20 | 45 | 40 |
| 113 | 20 | 110 | 90 |
| 114 | 15 | 85 | 70 |
| 115 | 110 | > 200 | 160 |
| 116 | 160 | 45 | 40 |
| 117 | 15 | 30 | 30 |
| 118 | 15 | 15 | 20 |
| 119 | 15 | 50 | 30 |
| 125 | 160 | 150 | 90 |
| 132 | > 200 | > 200 | > 200 |

| I.S. | IC50 | | |
|---|---|---|---|
| Immunosuppressant | | | |
| | MLR | aCD3 | aCD28 |
| CyA | 20 nM | 50 nM | N.S. |
| FK506 | 1 nM | 1 nM | N.S. |
| Rapamycin | 1 nM | 1 nM | 1 nM |
| Leflunomide | 25 μM | 15 μM | 20 μM |
| Mofetil | <0.5μM | 50 nM | 50 nM |

N.S. = not suppressive even not in the highest concentration

## Claims

**1.** Use of a xanthine derivative of general formula (I):

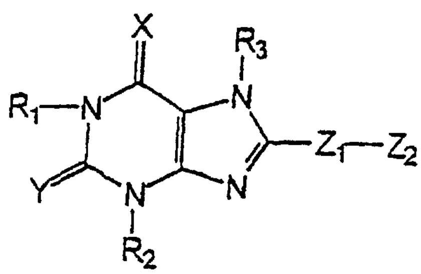

wherein:

$R_1$, $R_2$ and $R_3$ are independently hydrogen, saturated or unsaturated aliphatic chains which may be straight or branched having 1 to 6 carbon atoms;
X and Y are independently oxygen or sulfur;
$Z_1$ is selected from the group comprising a thienyl; furanyl; cyclopentyl, phenyl or a substituted by $Z_2$ or unsubstituted phenyl; wherein $Z_2$ is selected from the group comprising phenyl; sulfonic acid; unsubstituted or N-substituted sulfonamide with substituents such as alkyl, aminoalkyl where the amino group may be substituted itself with lower alkyl groups bearing 1 to 4 carbon atoms; nitro; halogen; substituted or unsubstituted amino group; aliphatic chain with 1 to 3 carbon atoms; halogenated aliphatic chain with 1 to 3 carbon atoms; aliphatic chain containing ether functions, acids, esters, amides, substituted or unsubstituted amines having 1 to 3 carbon atoms, nitro, sulfonamides or a combination of these functional groups with a maximum length of the chain of 12 atoms,

or a pharmaceutically acceptable salt thereof for the manufacture of a medicament for the treatment of autoimmuno disorders.

**2.** Use according to claim 1, wherein
$R_1$, $R_2$ and $R_3$ are independently hydrogen; saturated or unsaturated straight aliphatic chains having 1 to 3 carbon atoms; and
$Z_1$ is a substituted or unsubstituted phenyl.

**3.** Use according to claim 1 or 2, wherein the xanthine derivative is compound selected from the group comprising:

1,7-diallyl-3-methyl-8-fenylxanthine (8);
1,3-dipropyl-8-[4(dimethylamino(ethyl(amino(sulfonyl))))fenyl]xanthine (9);
1,3,7-trimethyl-8-(4-trifluoromethylfenyl)-xanthine (10);
1, 3-dipropyl-8- [4 (diethylamino (propyl (amino(sulfonyl))))fenyl]xanthine (11);
1,3-dipropyl-2-thio-8-[4-((((N-2-aminoethyl)-amino) carbonyl)methyl)oxy)fenyl]xanthine (35);
1,3-dipropyl-8-[4-dimethylamino(propyl(amino(sulfonyl))))fenyl]xanthine (40);
1,3-dimethyl-7-propyl-8-fenylxanthine (112);
1,7-diallyl-3-propyl-8-(p-bifenyl)xanthine (117);
1,3,7-tripropyl-8-(4-chlorofenyl)xanthine (118);
1,7-diallyl-3-propyl-8-(4-chlorofenyl)xanthine (119).

**4.** Product containing at least one compound according to any of the preceding claims 1-3 and a compound selected from the group comprising cyclosporine A, FK506, Rapamycin, Leflunomide, Mofetil.

**5.** Use of a product according to claim 4, as a combined preparation for simultaneous separate or sequential use in the treatment of auto-immuno disorders.

**6.** Compound having the formula:

1,7-dipropynyl-3-methylxanthine (2);
1-methyl-3,7-dipropynylxanthine (3);
1,3-dipropynyl-7-methylxanthine (4);
1,3,7-tripropynylxanthine (5);
1-(4-carboxybutyl)-3,7-dimethylxanthine (22);
1-(3-carboxypropyl)-3,7-dimethylxanthine (23);
1-(3-ethoxycarbonyl)propyl-3,7-dimethylxanthine (24);
1,7-dimethyl-3-propynylxanthine (67);
1,7-dimethyl-3-((<u>tert</u>butyl)methyl)xanthine (68);
1,3-dimethyl-7-allyl-8-[(4-trifluoromethyl)-fenyl]xanthine (82);
1,3-dimethyl-7-propyl-8-fenylxanthine (112);
1,3,7-tripropyl-8-fenylxanthine (113);
1,7-diallyl-3-propyl-8-fenylxanthine (114);
1,3,7-tripropyl-8-(p-bifenyl)xanthine (116);
1,7-diallyl-3-propyl-8-(p-bifenyl)xanthine (117);
1,3,7-tripropyl-8-(4-chlorofenyl)xanthine (118);
1,7-diallyl-3-propyl-8-(4-chlorofenyl)xanthine (119).

**7.** Compound having the formula:

1,7-dipropynyl-3-methylxanthine (2);
1-methyl-3,7-dipropynylxanthine (3);
1,3-dipropynyl-7-methylxanthine (4);
1,3,7-tripropynylxanthine (5).

**8.** Compound having the formula:

1-(4-carboxybutyl)-3,7-dimethylxanthine (22);
1-(3-carboxypropyl)-3,7-dimethylxanthine (23);
1-(3-ethoxycarbonyl)propyl-3,7-dimethylxanthine (24).

**9.** Compound having the formula:

1,3-dimethyl-7-allyl-8-[(4-trifluoromethyl)-fenyl]xanthine (82);
1,3-dimethyl-7-propyl-8-fenylxanthine (112);
1,3,7-tripropyl-8-fenylxanthine (113);
1,7-diallyl-3-propyl-8-fenylxanthine (114);
1,3,7-tripropyl-8- (p-bifenyl)xanthine (116).

**10.** Compound having the formula:

1,7-diallyl-3-propyl-8-(p-bifenyl) xanthine (117);
1,3,7-tripropyl-8-(4-chlorofenyl)xanthine (118);
1,7-diallyl-3-propyl-8-(4-chlorofenyl)xanthine (119).

**11.** Use of a compound according to any of the claims 6-10, as a medicament.

**12.** Use of 1-propynyl-3,7-dimethylxanthine or a compound of claims 6-10 for the manufacture of a medicament for the treatment of auto-immune disorders.

**Patentansprüche**

**1.** Verwendung eines Xanthin-Derivats der allgemeinen Formel (I):

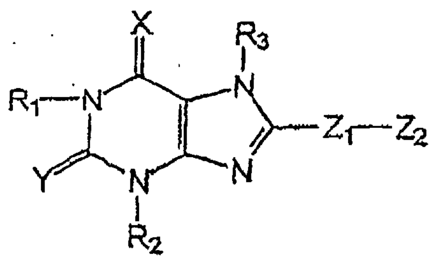

worin:

$R_1$, $R_2$ und $R_3$ unabhängig voneinander Wasserstoff oder gesättigte oder ungesättigte aliphathische Ketten sind, die geradkettig oder verzweigt sein können, mit 1 bis 6 Kohlenstoffatomen;
X und Y unabhängig voneinander Sauerstoff oder Schwefel sind;
$Z_1$ ausgewählt ist aus der Gruppe, die ein Thienyl; Furanyl; Cyclopentyl, Phenyl oder ein durch $Z_2$ substituiertes oder nicht-substituiertes Phenyl umfasst; wobei $Z_2$ ausgewählt ist aus der Gruppe, die Phenyl; Sulfonsäure; nicht-substituiertes oder N-substituiertes Sulfonamid mit Substituenten wie Alkyl und Aminoalkyl, wobei die Aminogruppe selbst substituiert sein kann mit Niedrigalkylgruppen, die 1 bis 4 Kohlenstoffatome tragen; Nitro; Halogen; eine substituierte oder nichtsubstituierte Aminogruppe; eine aliphatische Kette mit 1 bis 3 Kohlenstoffatomen; eine halogenierte aliphatische Kette mit 1 bis 3 Kohlenstoffatomen; eine aliphatische Kette, die Etherfunktionen, Säuren, Ester, Amide, substituierte oder nichtsubstituierte Amine mit 1 bis 3 Kohlenstoffatomen, Nitro, Sulfonamide oder eine Kombination dieser funktionellen Gruppen mit einer Maximallänge der Kette von 12 Atomen enthält; umfasst,

oder ein pharmazeutisch akzeptables Salz davon, zur Herstellung eines Medikaments zur Behandlung von Autoimmunerkrankungen.

**2.** Verwendung gemäß Anspruch 1, wobei
$R_1$, $R_2$ und $R_3$ unabhängig voneinander Wasserstoff; oder gesättigte oder ungesättigte, geradkettige aliphatische Ketten mit 1 bis 3 Kohlenstoffatomen sind; und
$Z_1$ ein substituiertes oder nicht-substituiertes Phenyl ist.

**3.** Verwendung gemäß Anspruch 1 oder 2, wobei das Xanthin-Derivat eine Verbindung ist, die aus der Gruppe ausgewählt ist, welche umfasst:

1,7-Diallyl-3-methyl-8-phenylxanthin (8);
1, 3-Dipropyl-8- [4(dimethylamino(ethyl(amino(sulfonyl))))-phenyl]xanthin (9) ;
1,3,7-Trimethyl-8-(4-trifluormethylphenyl)-Xanthin (10) ;
1,3-Dipropyl-8-[4(diethylamino(propyl(amino(sulfonyl))))-phenyl]xanthin (11) ;
1,3-Dipropyl-2-thio-8-[4-(((((N-2-aminoethyl)amino)-carbonyl)methyl)oxy)phenyl]xanthin (35);
1,3-Dipropyl-8-[4-dimethylamino(propyl(amino(sulfonyl)))-phenyl]xanthin (40);
1,3-Dimethyl-7-propyl-8-phenylxanthin (112);
1,7-Diallyl-3-propyl-8-(p-biphenyl)xanthin (117);
1,3,7-Tripropyl-8-(4-chlorphenyl)xanthin (118);
1,7-Diallyl-3-propyl-8-(4-chlorphenyl)xanthin (119).

**4.** Produkt, welches mindestens eine Verbindung gemäß irgendeinem der vorangehenden Ansprüche 1 bis 3 und eine Verbindung enthält, die aus der Gruppe ausgewählt ist, welche Cyclosporin A, FK506, Rapamycin, Leflunomid und Mofetil einschließt.

**5.** Verwendung eines Produkts gemäß Anspruch 4 als ein Kombinationspräparat zur gleichzeitigen, getrennten oder aufeinander folgenden Verwendung bei der Behandlung von Autoimmunerkrankungen.

**6.** Verbindung mit der Formel:

1,7-Dipropynyl-3-methylxanthin (2);
1-Methyl-3,7-dipropynylxanthin (3) ;
1,3-Dipropynyl-7-methylxanthin (4) ;
1,3,7-Tripropynylxanthin (5);
1-(4-Carboxybutyl)-3,7-dimethylxanthin (22);
1-(3-Carboxypropyl)-3,7-dimethylxanthin (23);
1-(3-Ethoxycarbonyl)propyl-3,7-dimethylxanthin (24) ;
1,7-Dimethyl-3-propylnylxanthin (67) ;
1,7-Dimethyl-3-((tertbutyl)methyl)xanthin (68);
1,3-Dimethyl-7-allyl-8-[(4-trifluormethyl)phenyl]xanthin (82) ;
1,3-Dimethyl-7-propyl-8-phenylxanthin (112) ;
1,3,7-Tripropyl-8-phenylxanthin (113) ;
1,7-Diallyl-3-propyl-8-phenylxanthin (114) ;
1,3,7-Tripropyl-8-(p-biphenyl)xanthin (116) ;
1,7-Diallyl-3-propyl-8-(p-biphenyl)xanthin (117) ;
1,3,7-Tripropyl-8-(4-chlorphenyl)xanthin (118) ;
1,7-diallyl-3-propyl-8-(4-chlorphenyl)xanthin (119).

**7.** Verbindung mit der Formel:

1,7-Dipropynyl-3-methylxanthin (2) ;
l-Methyl-3,7-dipropynylxanthin (3) ;
1,3-Dipropynyl-7-methylxanthin (4);
1,3,7-Tripropynylxanthin (5).

**8.** Verbindung mit der Formel:

1-(4-Carboxybutyl)-3,7-dimethylxanthin (22) ;
1-(3-Carboxypropyl)-3,7-dimethylxanthin (23) ;
1-(3-Ethoxycarbonyl)propyl-3,7-dimethylxanthin (24) ;

**9.** Verbindung mit der Formel:

1,3-Dimethyl-7-allyl-8-[(4-trifluormethyl)phenyl]xanthin (82) ;
1,3-Dimethyl-7-propyl-8-phenylxanthin (112) ;
1,3,7-Tripropyl-8-phenylxanthin (113);
1,7-Diallyl-3-propyl-8-phenylxanthin (114);
1,3,7-Tripropyl-8-(p-biphenyl)xanthin (116);

**10.** Verbindung mit der Formel:

1,7-Diallyl-3-propyl-8-(p-biphenyl)xanthin (117) ;
1,3,7-Tripropyl-8-(4-chlorphenyl)xanthin (118) ;
1,7-Diallyl-3-propyl-8-(4-chlorphenyl)xanthin (119) .

**11.** Verwendung einer Verbindung gemäß irgendeinem der Ansprüche 6 bis 10 als ein Medikament.

**12.** Verwendung von 1-Propynyl-3,7-dimethylxanthin oder einer Verbindung der Ansprüche 6 bis 10 zur Herstellung eines Medikaments zur Behandlung von Autoimmunerkrankungen.

## Revendications

**1.** Utilisation d'un dérivé de xanthine de formule générale (I) :

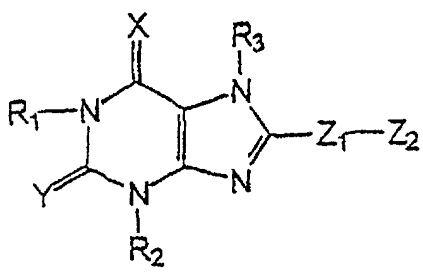

dans laquelle :

$R_1$, $R_2$ et $R_3$ représentent indépendamment un atome d'hydrogène, des chaînes aliphatiques saturées ou insaturées qui peuvent être linéaires ou ramifiées et comportant de 1 à 6 atomes de carbone ;
X et Y représentent indépendamment un atome d'oxygène ou de soufre ;
$Z_1$ est choisi parmi le groupe comprenant un groupe thiényle ; furannyle ; cyclopentyle, phényle ou un groupe phényle substitué par $Z_2$ ou non substitué ; $Z_2$ étant choisi parmi le groupe comprenant un groupe phényle ; un groupe acide sulfonique ; un groupe sulfonamide non substitué ou N-substitué avec des sustituants tels qu'un groupe alkyle et un groupe aminoalkyle, le groupe amino pouvant être lui-même substitué avec des groupes alkyle inférieur comportant de 1 à 4 atomes de carbone, un groupe nitro ; un atome d'halogène ; un groupe amino substitué ou non substitué ; une chaîne aliphatique comportant de 1 à 3 atomes de carbone ; une chaîne aliphatique halogénée comportant de 1 à 3 atomes de carbone ; une chaîne aliphatique contenant des fonctions éther, des groupes acides, des groupes ester, des groupe amide, des groupes amine substitués ou non substitués comportant de 1 à 3 atomes de carbone, des groupes nitro, des groupes sulfonamide ou une association de ces groupes fonctionnels, la longueur maximum de la chaîne étant de 12 atomes, ou
d'un sel pharmaceutiquement acceptable de celui-ci pour la préparation d'un médicament destiné au traitement d'affections auto-immunes.

**2.** Utilisation selon la revendication 1, dans laquelle
$R_1$, $R_2$ et $R_3$ représentent indépendamment des atomes d'hydrogène ; des chaînes aliphatiques linéaires saturées ou insaturées comportant de 1 à 3 atomes de carbone ; et
$Z_1$ représente un groupe phényle substitué ou non substitué.

**3.** Utilisation selon la revendication 1 ou 2, dans laquelle le dérivé de xanthine est un composé choisi parmi le groupe comprenant :

la 1,7-diallyl-3-méthyl-8-phénylxanthine (8) ;
la 1,3-dipropyl-8-[4(diméthylamino(éthyl(aminosulfonyl))))phényl]xanthine (9) ;
la 1,3,7-triméthyl-8-(4-trifluorométhylphényl)-xanthine (10) ;
la 1,3-dipropyl-8-[4(diéthylamino(propyl(amino(sulfonyl))))phényl]xanthine (11) ;

la 1,3-dipropyl-2-thio-8-[4-(((N-2-aminoéthyl)-amino)carbonyl)méthyl)oxy)phényl]xanthine (35) ;
la 1,3-dipropyl-8-[4-diméthylamino(propyl(amino(sulfonyl))))phényl]xanthine (40) ;
la 1,3-diméthyl-7-propyl-8-phénylxanthine (112) ;
la 1,7-diallyl-3-propyl-8-(p-biphényl)xanthine (117) ;
la 1,3,7-tripropyl-8-(4-chlorophényl)xanthine (118) ;
la 1,7-diallyl-3-propyl-8-(4-chlorophényl)-xanthine (119).

**4.** Produit contenant au moins composé selon l'une quelconque des revendications précédentes 1 à 3, et un composé choisi parmi le groupe comprenant la ciclosporine A, le FK506, la rapamycine, le léflunomide, le mofétil.

**5.** Utilisation d'un produit selon la revendication 4 sous forme d'une composition pour une utilisation simultanée, séparée ou successive dans le traitement d'affections auto-immunes.

**6.** Composé de formule :

1,7-dipropynyl-3-méthylxanthine (2) ;
1-méthyl-3,7-dipropynylxanthine (3) ;
1,3-dipropynyl-7-méthylxanthine (4) ;
1,3,7-tripropynylxanthine (5) ;
1-(4-carboxybutyl)-3,7-diméthylxanthine (22) ;
1-(3-carboxypropyl)-3,7-diméthylxanthine (23) ;
1-(3-éthoxycarbonyl)propyl-3,7-diméthylxanthine (24) ;
1,7-diméthyl-3-propynylxanthine (67) ;
1,7-diméthyl-3-((tertbutyl)méthyl)xanthine (68) ;
1,3-diméthyl-7-allyl-8-[(4-trifluorométhyl)-phényl]xanthine (82) ;
1,3-diméthyl-7-propyl-8-phénylxanthine (112) ;
1,3,7-tripropyl-8-phénylxanthine (113) ;
1,7-diallyl-3-propyl-8-phénylxanthine (114) ;
1,3,7-tripropyl-8-(p-biphényl)xanthine (116) ;
1,7-diallyl-3-propyl-8-(p-biphényl)xanthine (117) ;
1,3,7-tripropyl-8-(4-chlorophényl)xanthine (118) ;
1,7-diallyl-3-propyl-8-(4-chlorophényl)xanthine (119).

**7.** Composé de formule :

1,7-dipropynyl-3-méthylxanthine (2) ;
1-méthyl-3,7-dipropynylxanthine (3) ;
1,3-dipropynyl-7-méthylxanthine (4) ;
1,3,7-tripropynylxanthine (5).

**8.** Composé de formule :

1-(4-carboxybutyl)-3,7-diméthylxanthine (22) ;
1-(3-carboxypropyl)-3,7-diméthylxanthine (23) ;
1-(3-éthoxycarbonyl)propyl-3,7-diméthylxanthine (24).

**9.** Composé de formule :

1,3-diméthyl-7-allyl-8-[(4-trifluorométhyl)-phényl]xanthine (82) ;
1,3-diméthyl-7-propyl-8-phénylxanthine (112) ;
1,3,7-tripropyl-8-phénylxanthine (113) ;
1,7-diallyl-3-propyl-8-phénylxanthine (114) ;
1,3,7-tripropyl-8-(p-biphényl)xanthine (116).

**10.** Composé de formule :

1,7-diallyl-3-propyl-8-(p-biphényl)xanthine (117) ;
1,3,7-tripropyl-8-(4-chlorophényl)xanthine (118) ;

1,7-diallyl-3-propyl-8-(4-chlorophényl)xanthine (119).

**11.** Utilisation d'un composé selon l'une quelconque des revendications 6 à 10 comme médicament.

**12.** Utilisation de la 1-propynyl-3,7-diméthylxanthine ou d'un composé des revendications 6 à 10 pour la préparation d'un médicament destiné au traitement des affections auto-immunes.